(19) Europäisches Patentamt
European Patent Office
Office européen des brevets

(11) Publication number: 0 266 580 A2

# (12) EUROPEAN PATENT APPLICATION

(21) Application number: 87114860.7

(51) Int. Cl.[4]: B01J 20/32 , G01N 33/548

(22) Date of filing: 12.10.87

(30) Priority: 03.11.86 SE 8604684

(43) Date of publication of application: 11.05.88 Bulletin 88/19

(84) Designated Contracting States: BE CH DE ES FR GB IT LI NL

(71) Applicant: EXCORIM KB
Box 10101
S-220 10 Lund(SE)

(72) Inventor: Larsson, Per-Olof
Fagelhundsvägen 56
S-222 53 Lund(SE)
Inventor: Johnson, Kersti Barbro
Fagottgränden 21 B
S-223 68 Lund(SE)
Inventor: Nylén, Ulf Thomas Gustav
Borgarevägen 15
S-222 47 Lund(SE)
Inventor: Wikström, Per Ingvar Oskar
Flöjtvägen 22a
S-223 68 Lund(SE)
Inventor: Zetterstrand, Ingrid Kristina
Kattesund 8
S-222 23 Lund(SE)

(74) Representative: Boberg, Nils Gunnar Erik
Gambro AB Patent Department Box 10101
S-220 10 Lund(SE)

## (54) A method for coating solid particles with a hydrophilic gel and particles coated by the method.

(57) A method for coating solid particles with a hydrophilic gel, characterized in that as the said particles hydrophilic particles are chosen which are mixed with a gel-forming substance at a temperature which is above the gelling temperature. The gel-forming substance is made to cover each individual particle, whereafter the particles are separated from each other and cooled to a temperature which is below the gelling temperature.

The invention also relates to particles coated in the above-mentioned manner.

The gel-coated particles so obtained either may be used as such in various separating processes, e g as ion exchanger, if the gel-forming substance contains charged groups. Alternatively the gel-coated particles subsequently may be provided with suitable adsorbent groups, e g ion exchange groups, hydrophobic groups or groups with biospecificity. Examples of such groups are enzyme inhibitors, enzymes, antibodies and protein A from staphylococcus Aureus.

EP 0 266 580 A2

# A METHOD FOR COATING SOLID PARTICLES WITH A HYDROPHILIC GEL AND PARTICLES COATED BY THE METHOD

## TECHNICAL FIELD

In chemistry using chromatography in general and in immunoadsorption therapy in particular there is a great need for various particles suitable as supports for different adsorbent groups. Especially desirable in this context are supports with a large active surface which rapidly and effectively can be brought into equilibrium with a flowing medium and offer a low flow resistance. A support which presents the above-mentioned properties may consist of a solid inner core with only small pores or none at all, coated with a thin layer of a gel intended as a bond for the above-mentiond adsorbent groups. It is the object of the present invention, therefore, to provide a method for the manufacture of such a support and, more particularly, a core coated with a hydrophilic gel.

## BACKGROUND ART

Columns containing solid supports with immuno-adsorbents immobilized thereon are found, for example, in the American patents 4 180 383 and 4 215 688. In at least the firstnamed of these patents a support is described in detail which consists of an inner solid core with one or more outer layers of active material. The description of how these outer layers are produced, however, is very inadequate.

## DISCLOSURE OF INVENTION

In accordance with the invention a method is thus provided for the coating of solid particles with a hydrophilic gel. The method in accordance with the invention in short can be said to be based on the generally known principles that hydrophilic substances and hydrophilic particles are drawn towards one another, at the same time as a rejection effect exists in relation to hydrophobic substances. Use is made of this in the method according to the invention, which is characterized in that as the said particles hydrophilic particles are chosen which are so mixed with a gel-forming substance at a temperature, which is above the gelling temperature of the substance, that the gel-forming substance is made to cover each individual particle, whereupon the particles are separated from each other and cooled to a temperature which is below the gelling temperature. As is evident from the following, the mixing as well as the separation, may take place in many different ways.

In accordance with a preferred embodiment the separation takes place in that the coated particles are dispersed in a hydrophobic solvent which subsequently is cooled. This may be carried out, for example, with vigorous and effective stirring, which first separates possibly cohering particles and then keeps the particles separate. It has been found that the hydrophilic gel-forming substance is drawn towards the individual hydrophilic particles and deposits as a thin layer around the same. On cooling the gel-forming substance naturally solidifies, whereafter the coated particles can be readily separated from the hydrophobic solvent.

The dispersion is facilitated especially through an addition of a dispersing agent, e g a soap with a hydrophilic end and a hydrophobic end. Because of such a structure this agent automatically settles in the bundary layer between the hydrophilic gel and the hydrophobic solvent.

The dispersion is facilitated further if the said particles, the gel-forming substance and the hydrophobic solvent are selected so that their density is substantially identical. It is also possible, though, to use particles and/or gel-forming substances of a density other, for example higher, than that of the hydrophobic solvent. In such a case, however, it is necessary to select as the said hydrophobic solvent one of a high viscosity, so that the particles with the gel-forming substance appended thereto are prevented from rising or sinking too fast, and they are likewise prevented from making contact with one another which may lead to clotting.

The mixing of the gel-forming substance with the particles preferably is done separately before the dispersion in the hydrophobic solvent. However, it may also be done in this solvent.

The separation, as mentioned above, may thus be carried out through vigorous stirring of the coated particles in a solvent. Alternatively it may be brought about instead by forcing the particles mixed with the gel-forming substance through a sieve or the like of such a mesh size that only one coated particle at a time can pass the respective holes in the same. Such a sifting too has made it evident that the coating on the various particles becomes very even.

From the sieve the separated particles can be made to drop down freely into a cooling agent and/or solvent. The simplest and least expensive that may be used here is a cooling agent which consists of water of a temperature below the gelling temperature. It has been found, however, that a somewhat more uniform coating can be achieved if instead a hydrophobic solvent is used in the same manner as above. The surface temperature of this solvent may be made to exceed the gelling temperature, whilst at the same time its bottom temperature can be kept below the same. In this way a uniform distribution of the gel-forming layer can be obtained at the same time as the particles sink down to the region where the gel itself solidifies.

As material for the particles preferably a hydrophilic glass is chosen. However, other materials too, e g from the group PVC, polyamide and polycarbonate, may be chosen. In such a case, though, the particles have to be pretreated first so as to achieve a hydrophilic surface.

The particle size chosen is appropriately between 0.001 and 5 mm, preferably between 0.15 and 1 mm.

As an example of a suitable gel agarose may be mentioned. For this material it has been found appropriate to choose a thickness of the gel layer between 0.0001 and 1.0 mm, preferably between 0.001 and 0.04 mm. Alternatively a gel from the group agar, cappacarrageenan, starch and chitosan may be selected.

After the gelling the coated particles are separated from the cooling agent and/or solvent used. Thereafter the gel may be washed and possibly strengthened, e g through cross-linkage.

The gel-coated particles so obtained either may be used as such in various separating processes, e g as ion exchanger, if the gel-forming substance contains charged groups. Alternatively the gel-coated particles subsequently may be provided with suitable adsorbent groups, e g ion exchange groups, hydrophobic groups or groups with biospecificity. Examples of such groups are enzyme inhibitors, enzymes, antibodies and protein A from staphylococcus Aureus.

The invention also relates to coated particles, characterized in that they are manufactured in accordance with the method of anyone of the subsequent claims.

EXAMPLE 1

Agarose covering of glass beads in paraffin oil

Material:

150 ml paraffin oil
1.2 g sorbitan sesquioleate
6.0 g glass beads approx. 0.2 mm diameter
3.0 ml 3% agarose of gelling temperature below 30°C.

Execution:

1. The paraffin oil was heated with the above-mentioned sesquioleate to 40-45°C.

2. The agarose was melted in the water which was heated to boiling point, and was mixed thereafter with the glass beads at 45°C, whereupon the mixture was added to the oil, with stirring (750 rpm).

3. The stirring was continued at the said temperature of 40-45°C for 5 minutes. Thereafter it is cooled for 5 minutes in ice water with continued stirring, and this is continued for a further 10 minutes, but now essentially at room temperature.

4. Finally the coated glass beads were separated from the paraffin oil and washed on a coarse filter (0.1 mm) with small portions of ether and water.

Result Well coated glass beads with a layer thickness of 5-15 µ.

EXAMPLE 2

Agarose covering of dextran (Sephadex G® 25 coarse) in n-butanol

Material:

150 ml n-butanol
2 g Sephadex® G 25 coarse
10 ml 0.5 agarose of gelling temperature below 30°C.

Execution:

1. The n-butanol was heated on the water bath to approx. 50°C.

2. The dextran was measured and allowed to swell in approx. 20 ml distilled water. The excess water was removed by suction through a glass filter funnel.

3. The agarose was heated in the water until it had melted, and was then mixed with the dextran at approx. 40°C.

4. The mixture was added to the n-butanol, and was stirred for 5 minutes at 40°C.

5. The water bath was removed and the n-butanol was allowed to assume room temperature while stirring was continued.

6. Finally the gel was washed on a glass filter funnel with 50% acetone and distilled water.

Result

Well coated dextran particles with a layer thickness of 15-30 µ.

EXAMPLE 3

Coating of agarose on glass beads of 0.5 mm diameter

0.5 mm glass beads were boiled under reflux with 5% $HNO_3$ during 10 minutes and washed thereafter with distilled water. They were then dried overnight at a temperature of 170°C.

3% agarose (Sea plaque® from FMC Corp) of a gelling temperature below 30°C was placed in a heating chamber at a temperature of 41°C.

The glass beads (40) ml and agarose (4 ml) were mixed in a 200 ml round-bottom flask with ground-glass stopper. Everything had been preheated to 41°C. The mixing was carried out during 3 minutes by shaking the flask vigorously by hand. The flask was then maintained at 41°C with continued shaking for 10 minutes, whereupon the shaking was stopped and the glass beads were poured out onto a strainer with 0.8 mm holes. The strainer had also been heated to 41°C. The straining too was carried out, therefore, at this temperature. The glass beads with the adsorbed agarose were forced through the strainer with the help of a brush. They were allowed to fall down directly into a 5 1 beaker which was halffilled with ice-cold water.

Result

The glass beads surrounded by solidified agarose collected at the bottom of the beaker, the layer thickness being 4-10µ. A relatively even coating was obtained.

EXAMPLE 4

Cross-linkage of agarose-coated glass beads

Material:

6.0 g agarose-coated glass beads (0.2 mm in diameter)
6.0 ml 1 m NaOH with
30 mg $NaBH_4$
0.6 ml epichlorhydrin

Execution:

1. The agarose-coated glass beads were washed with distilled water and a quantity of 6.0 g was weighed. The measured quantity was suspended in a little distilled water.

2. NaOH with $NaBH_4$ was added followd by epichlorhydrin.

3. The mixture took place in a tube which was rocked very gently overnight with one turn approximately every twenty minutes.

4. The gel was washed with distilled water until all the NaOH and possibly remaining epichlorhydrin had been removed. Then neutralization with acetic acid was carried out.

Result

Substantially strengthened gel-layer.

EXAMPLE 5

Coating of agarose on polyamide particles

Polyamide-6 particles of a diameter of 0.5 mm were washed with a detergent (Duponol R A® ) in alkaline medium. The polyamide subsequently was weakly hydrolyzed with 3.65 m HCl at elevated temperature. Then the polyamide was washed with distilled water and dried. 900 mg agarose (Sea Kem ME® from FMC Corp) was suspended in 30 ml distilled water and boiled under reflux for 10 minutes.

150 ml toluene, 50 ml carbon tetrachloride and 0.3 sorbitan sesquiolate were heated to 50°C.

40 ml polyamide granules were added to the agarose (note that alternatively the polyamide granules may be poured into the organic solvent first and the agarose then added to the suspension of polyamide in toluene-carbon tetrachloride).

The agarose with polyamide was poured into the organic solvent and stirred at a stirring rate of 1500 rpm. The stirring was allowed to continue for 5 minutes, whereafter the suspension was cooled with continued stirring. The cooling was performed by placing the beaker in an ice-bath.

When the solution had cooled down to room temperature the excess solution was filtered off through a glass filter.

The gel finally was washed on the glass filter with toluene, diethyl and water.

Result

From a microscopic examination of the gel it was evident that each polyamide particle had acquired a thin layer of agarose of a thickness of 15-30 µ.

## SUPPLEMENTARY EXPLANATIONS

If organic solvent and water are used as described above, they should have a low mutual solubility, which implies that they form two phases.

If the substances included in the operation are of the same density and a water-soluble gel is used, the density of this gel-forming substance in liquid state may be adjusted through the addition of a salt. Thus the density may be adjusted e g with the help of potassium iodide in the range 1.0 g per $cm^3$ to 1.7 g per $cm^3$ for the gelling water phase.

The density of the hydrophobic phase can be adjusted by mixtures of e g toluene and carbon tetrachloride in the range 0.9 to 1.6 g per $cm^3$. If still higher density is required, bromoform can be used instead of carbon tetrachloride. Most plastic materials (polyamide, polycarbonate, polystyrene, PVC, acrylate etc) have a density in the range between 1 g per $cm^3$ and 1.6 g per $cm^3$. Inorganic material, such as e g glass or coal, on the other hand, have a considerably higher density.

Many plastic materials are hydrophobic by their nature. However, most plastics can be rendered hydrophilic on their surface by means of modification of the surface, so that the surface layer becomes hydrophilic. Such a modification can be carried out in many different ways. The most usual, though, is to subject the material to strong acids, oxizing agent, radiation or strong bases in a first step. Sometimes this treatment is sufficient for making the surface hydrophilic. In other cases, simultaneously with the above-mentioned agressive treatment some substance may be present which bonds to the surface and renders it hydrophilic.

## ALTERNATIVE STRENGTHENING OF THE GEL LAYER

Chemical cross-linkages can be obtained in that bifunctional reagents are made to bond together the matrix. The majority of hydrophilic gels contain free hydroxyl groups. Cross-linkage between free hydroxyl groups can be established, for example, by means of:

Epichlorhydrin 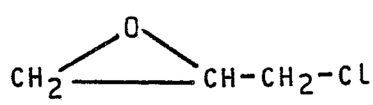

2-3 dibromopropanol $Br-ChH_2-CHBr-CH_2OH$

Diphenyl sulphon $CH_2 = CH-SO_2-CH = CH_2$

An alternative method for arranging cross-linkage consists in manufacturing a gel which in itself contains a quantity of reactive groups. These reactive groups can be obtained, for example, by oxidizing the hydroxyl groups to aldehyde groups. A variant of this is to use gel-forming substances which from the start contain a number of active groups. An example of this is glyoxyl agarose which contains free aldehyde groups.

After the glyoxylagarose with free aldehyde groups has been made to solidify, the gel simply can be cross-linked by the addition of a diamino compound $H_2N-CH_2-R-CH_2-NH_2$, where R = $(CH_2)n$ and where n = 0-10, whereafter the Schiff bases formed are reduced with e g sodium borohydride $NaBH_4$.

Naturally the invention is not limited to the examples described above, but may be varied within the scope of the following claims. For example, the invention may be applied to many other gels, solvents/cooling agents and particles respectively.

## Claims

1. A method for coating solid particles with a hydrophilic gel, **characterized** in that as the said particles hydrophilic particles are chosen which are so mixed with a gel-forming substance at a temperature, which is above the gelling temperature, that the gel-forming substance is made to cover each individual particle, whereupon the particles are separated from each other and cooled to a temperature which is below the gelling temperature.

2. A method in accordance with claim 1, **characterized** in that the separation takes place by dispersing the coated particles in a hydrophobic solvent which subsequently is cooled.

3. A method in accordance with claim 2, **characterized** in that the dispersion is facilitated through the addition of a dispersing agent.

4. A method in accordance with claim 2 and 3, **characterized** in that the particles, the gel and the said hydrophobic solvent are selected so that their density is substantially the same.

5. A method in accordance with claim 2 and 3 using particles and/or gel of a density other, in particular higher, than that of the hydrophobic solvent, **characterized** in that as the said hydrophobic solvent one of a high viscosity is selected.

6. A method in accordance with anyone of claims 2-5, **characterized** in that the mixing takes place in the hydrophobic solvent.

7. A method in accordance with anyone of claims 2-5, **characterized** in that the particles are mixed with the gel-forming substance before the mixture is dispersed in the hydrophobic solvent.

8. A method in accordance with claim 1, **characterized** in that the separation is brought about mechanically in that the particles mixed with the gel are forced through a sieve or the like of such a mesh size that only one coated particle at a time can pass the respective holes in the same.

9. A method in accordance with claim 8, **characterized** in that the separated particles are allowed to drop down freely from the sieve into cold air, a cold gas or another cooling agent and/or solvent.

10. A method in accordance with claim 9, **characterized** in that the said cooling agent consists of water of a temperature below the gelling temperature.

11. A method in accordance with claim 9, **characterized** in that the said solvent consists of a hydrophobic one.

12. A method in accordance with claim 11, **characterized** in that the surface temperature of the hydrophobic solvent exceeds the gelling temperature whilst its bottom temperature is below the same.

13. A method in accordance with anyone of the preceding claims, **characterized** in that as the said particles hydrophilic glass particles are chosen.

14. A method in accordance with anyone of claims 1-12, **characterized** in that as the material for the said particles one from the group of PVC, polyamide, acrylate, methylmethacrylate, polystyrene and polycarbonate is chosen, the particles being pretreated first so as to produce a hydrophilic surface.

15. A method in accordance with anyone of the preceding claims, **characterized** in that the particle size is chosen between 0.001 and 5 mm, preferably between 0.15 and 1 mm.

16. A method in accordance with anyone of the preceding claims, **characterized** in that as the gel one within the group of agarose, agar, cappa carrageenan, starch and chitosan, preferably agarose, is chosen.

17. A method in accordance with claim 16, **characterized** in that for the gel layer a thickness between 0.0001 and 1.0 mm, preferably between 0.001 and 0.05 mm is chosen.

18. A method in accordance with anyone of the preceding claims, **characterized** in that the particles with gel layer are washed so as to be strengthened thereafter, preferably through cross-linkage.

19. A method in accordance with anyone of the preceding claims, **characterized** in that adsorbent units are bonded chemically to the gel, e g ion exchange groups, hydrophobic groups or groups units with biospecificity, e g enzyme inhibitors, enzymes, antigens, protein A or antibodies.

20. Coated particles, **characterized** in that they are manufactured in accordance with the method of anyone of the preceding claims.